# EUROPEAN PATENT SPECIFICATION

(11) **EP 2 574 297 B1**
(45) Date of publication and mention of the grant of the patent: **11.11.2015**
(21) Application number: 11183616.9
(22) Date of filing: 30.09.2011
(51) Int. Cl.: A61B 17/70

(54) **Bone anchoring device and tool cooperating with such a bone anchoring device**
Knochenverankerungsvorrichtung und Werkzeug, das mit der Knochenverankerungsvorrichtung arbeitet
Dispositif d'ancrage d'os et outil fonctionnant avec un tel dispositif d'ancrage d'os

(43) Date of publication of application: 03.04.2013
(73) Proprietor: Biedermann Technologies GmbH & Co. KG, 78166 Donaueschingen (DE)
(72) Inventor: Biedermann, Lutz, 78048 VS-Villingen (DE); Biedermann, Timo, 78647 Trossingen (DE); Matthis, Wilfried, 79367 Weisweil (DE); Dannecker, Berthold, 78112 St. Georgen (DE)
(74) Representative: Prüfer & Partner GbR European Patent Attorneys

(56) References cited:
- WO-A1-2011/043799
- US-A1- 2004 254 576
- US-A1- 2005 096 653
- US-A1- 2006 074 445
- US-A1- 2006 173 454
- US-A1- 2009 149 887
- US-A1- 2010 160 977
- US-A1- 2010 204 735
- US-B1- 6 273 888
- US-B1- 6 743 231

## Description

The invention relates to a bone anchoring device including a bone anchoring element and a receiving part for coupling a rod to the bone anchoring element. The receiving part comprises a rod receiving portion for receiving the rod and a head receiving portion that is flexible so as to allow introduction and clamping of the head. The bone anchoring device further comprises a locking ring arranged around the head receiving portion that can assume a locking position in which the head is locked by compression of the head receiving portion of the receiving part. The locking ring has an outer surface portion with engagement portions for engagement with a tool. The tool is configured to cooperate with the receiving part and the locking ring so as to allow releasing of the locking ring from the position in which it locks the head. The bone anchoring device can be realized, for example, in the form of a polyaxial bone screw.

US 2009/0149887 A1 discloses an apparatus for connecting a bone anchor to a support rod, the apparatus including a connector body and a cap. The connector body has a socket for insertion, angulation and removal of a bone anchor. A sleeve is provided, which is configured to fit over the connector body in a temporary position, in which the sleeve permits insertion of the bone anchor, to move to a provisional locking position, in which the sleeve permits angulation but prevents removal of the bone anchor, and to move to a locking position in which the sleeve prevents both angulation and removal of the bone anchor. Tools are provided for installing the connector body, the sleeve, a cap and the support rod.

US 2010/204735 A1 discloses a coupling assembly with a two-part yoke device that allows the relative orientation between an anchor member and an elongate member to be widely varied.

Furthermore, US 2005/096653 A1 discloses a low profile ortho-pedic device for fixing and stabilizing bones to correct anomalies in skeletal structure. Clamps are provided which are movably attached to the screws with each clamp including a compression ring. A connecting rod connects several screws through slots in the clamps. The clamps are tightened to hold the rod and the heads in a pre-selected position by linear movement of the compression rings.

It is the object of the invention to provide a bone anchoring device that makes use of an outer locking ring for compressing the head receiving portion to lock the head and that is improved with respect to its handling. Further, a tool shall be provided that allows the improved handling.

The object is solved by a bone anchoring device according to claim 1 and by a system comprising a tool and a bone anchoring device according to claim 9. Further developments are given in the dependent claims.

The bone anchoring device allows a safe loosening of the locking of the head. Also, the bone anchoring device enables the surgeon to carry out revisions of the angular positioning of the receiving part with respect to the bone anchoring element. In one embodiment, the releasing of the locking is possible with the rod still inserted.

The tool is configured to engage the receiving part and confirmed to be operated along a central axis of the receiving part. Therefore, it is not necessary to have additional space for laterally applying the tool. Furthermore, the tool will not be jammed by the action of forces acting only from one side.

The handling of the bone anchoring device is simplified, because once a locking of the head is achieved, such locking can be released without applying large forces that could result in damage of surrounding material, such as tissue, blood vessels or nerves. Revisions or secondary adjustments of the rod and the receiving part can be performed in a controlled manner.

With the bone anchoring device, a modular system can be provided that allows to combine various anchoring elements with any suitable receiving part on demand depending on the actual clinical requirements. This reduces the costs of polyaxial screws, reduces the inventory and gives the surgeon a substantial choice of implants.

Further features and advantages of the invention will become apparent from the description of embodiments by means of the accompanying drawings.

In the drawings:
- Fig. 1:: shows a perspective exploded view of a first embodiment of the bone anchoring device.
- Fig. 2:: shows a perspective view of the bone anchoring device of Fig. 1 in an assembled state.
- Fig. 3:: shows an enlarged side view of the bone anchoring device in the assembled state.
- Fig. 4:: shows a cross-sectional view of the bone anchoring device shown in Fig. 3, the section being taken perpendicular to the rod axis.
- Fig. 5:: shows a perspective view of the receiving part.
- Fig. 6:: shows a cross-sectional view of the receiving part of Fig. 5, the section taken perpendicular to the channel axis for receiving the rod.
- Fig. 7:: shows a top view of the receiving part of Fig. 5.
- Fig. 8:: shows a perspective view of the locking ring of the bone anchoring device according to the first embodiment.
- Fig. 9:: shows a cross-sectional view of the locking ring, the section taken perpendicular to the rod channel.
- Fig. 10:: shows a top view of the locking ring of Fig. 8.
- Fig. 11:: shows a perspective exploded view of a tool according to a first embodiment that cooperates with the bone anchoring device according to the first embodiment.
- Fig. 12:: shows a cross-sectional view of the tool according to Fig. 11 in an enlarged view, wherein the section is taken through the central axis and perpendicular to the long side of a handle portion.
- Fig. 13:: shows a perspective view of a first step of mounting the tool to the bone anchoring device.
- Fig. 14:: shows a cross-sectional view of a further step of mounting the tool according to the first embodiment to the bone anchoring device according to the first embodiment.
- Fig. 15:: shows a perspective view of a next step of mounting the tool.
- Fig. 16:: shows an enlarged cross-sectional view of a portion of the tool attached to the bone anchoring device shown in Fig. 15.
- Fig. 17:: shows a perspective view of a further step of mounting the tool to the bone anchoring device.
- Fig. 18:: shows an enlarged cross-sectional view of a portion of the tool mounted to the bone anchoring device as shown in Fig. 17.
- Fig. 19:: shows a further enlarged portion of Fig. 18.
- Fig. 20:: shows a perspective view of a first step of releasing the locking of the head with the tool.
- Fig. 21:: shows an enlarged cross-sectional view of the tool and bone anchoring device shown in Fig. 20.
- Fig. 22:: shows an enlarged portion of Fig. 21.
- Fig. 23:: shows a perspective view of a step of removing the tool.
- Fig. 24:: shows an enlarged cross-sectional view of a portion of the tool with bone anchoring device shown in Fig. 23.
- Fig. 25:: shows a perspective view of a bone anchoring device according to a second embodiment in an assembled state.
- Fig. 26:: shows a side view of the locking ring of the bone anchoring device according to the second embodiment shown in Fig. 25.
- Fig. 27:: shows a top view of the locking ring of Fig. 26.
- Fig. 28:: shows a cross-sectional view of the locking ring of Fig. 26.
- Fig. 29:: shows a side view of the tool according to the second embodiment.
- Fig. 30:: shows a cross-sectional view of the tool shown in Fig. 29, the section taken along the central axis and perpendicular to the long side of the handle portion of the tool.
- Fig. 31:: shows a perspective view of a first step of mounting the tool according to the second embodiment to the bone anchoring device.
- Fig. 32:: shows a perspective view of a next step of mounting the tool to the bone anchoring device.
- Fig. 33:: shows a further step during the procedure of mounting the tool to the bone anchoring device.
- Fig. 34:: shows a perspective view of a next step of mounting, wherein the ribs at the locking ring are engaged by the tool.
- Fig. 35:: shows a cross-sectional view of the bone anchoring device according to the second embodiment with the tool according to the second embodiment mounted thereon.
- Fig. 36:: shows an enlarged cross-sectional view of the device of Fig. 35 rotated by around 45°.
- Fig. 37:: shows an enlarged portion of Fig. 36.
- Fig. 38:: shows a cross-sectional view of the bone anchoring device according to the second embodiment in a step removing the tool according to the second embodiment.

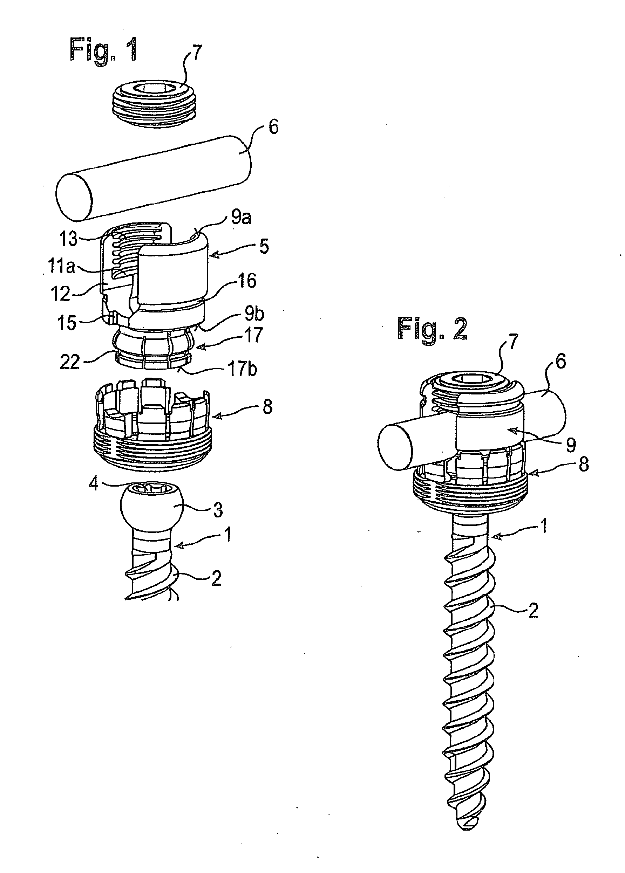

As shown in Figs. 1 and 2, the bone anchoring device comprises a bone anchoring element 1 in the form of a bone screw having a shank 2 with a threaded portion and a head 3 with a spherically-shaped outer surface portion. The head 3 has a recess 4 for engagement with a driver. The bone anchoring device also comprises a receiving part 5 for receiving a rod 6 to be connected to the bone anchoring element 1. Further, a fixation element 7 in the form of an inner screw is provided for fixing the rod 6 in the receiving part 5. The bone anchoring device includes a locking ring 8 for locking the head 3 in the receiving part 5.

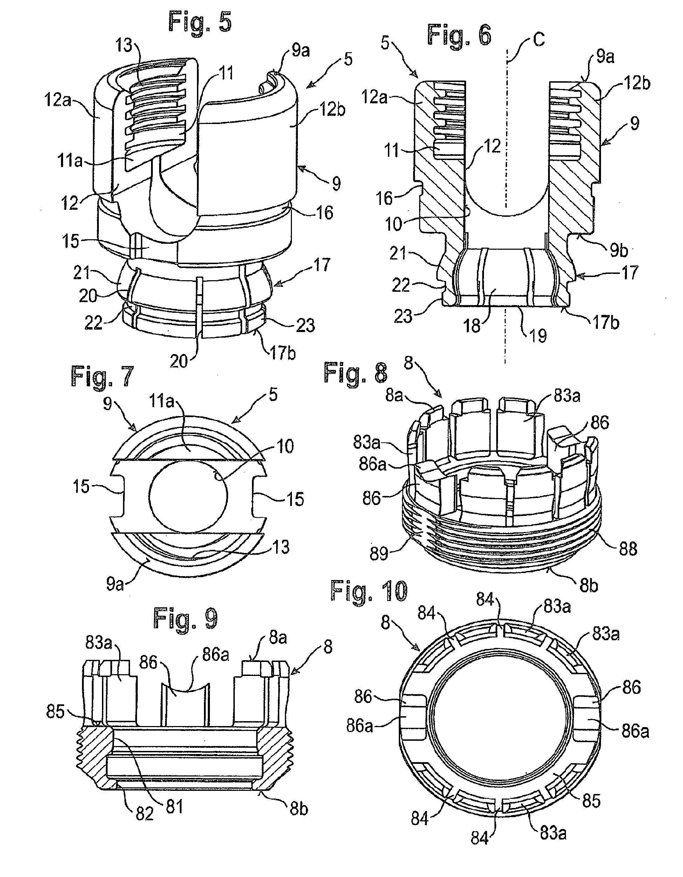

Referring to Figs. 1 to 7, the receiving part 5 comprises a rod receiving portion 9, that is substantially cylindrical and has a first end 9a and an opposite second end 9b and an axis of symmetry C that passes through the first end 9a and the second end 9b of the rod receiving portion. A coaxial first bore 10 is provided at the second end 9b as shown, for example, in Figs. 6 and 7. The diameter of the first bore 10 is smaller than the diameter of the head 3 of the bone anchoring element. The rod receiving portion 9 further includes a coaxial second bore 11 extending from the first end 9a to a distance from the second end 9b. The diameter of the second bore 11 is larger than the diameter of the first bore 10. By the second bore 11 an abutment surface 11a is provided inside the rod receiving portion that may serve as abutment for a tool to be described below. A substantially U-shaped recess 12 extends from the first end 9a in the direction of the second end 9b in the rod receiving portion 9, wherein the diameter of the recess 12 is slightly larger than the diameter of the rod 6 in such a way that the rod 6 can be placed in the recess and can be guided therein. By means of the recess 12, two free legs 12a, 12b are formed on which an internal thread 13 is provided. The internal thread can be a metric thread, a flat thread, a negative angle-thread, a saw-tooth thread, or any other thread form. Preferably, a thread form such as a flat thread or a negative angle thread is used that prevents splaying of the legs 12a, 12b, when the inner screw 7 is screwed-in. The depth of the recess 12, that forms a channel for the rod 6 is such that the rod 6, and the inner screw 7 can be inserted between the legs. Cut-outs 15 are provided in the rod receiving portion 9 extending from the second end 9b up to the recess 12. The cut-outs 15 are provided on either end of the channel formed by the recess 12.

The inner screw 7 has a thread corresponding to the internal thread 13 provided on the legs 12a, 12b. If a thread form that prevents the legs from splaying is used, a single locking element such as the inner screw 7 is sufficient.

In the outer surface of the rod receiving portion 9 in the region of the legs 12a, 12b a groove 16 is provided that extends in a circumferential direction and serves for engagement with a portion of the locking ring 8. The groove 16 is asymmetric in such a way that it allows a disengagement of the locking ring 8 and the groove when the locking ring 8 is shifted downwards away from the second end 9b.

At the side of the second end 9b, the receiving part further comprises a head receiving portion 17 providing an accommodation space for the head 3 of the bone anchoring element 1. The head receiving portion 17 has a greatest outer diameter that is smaller than the greatest outer diameter of the rod receiving portion 9. An internal hollow section 18 forms a seat for the head 3 of the bone anchoring element 1 and is open via the opening 19 to a free end 17b of the head receiving portion 17. The internal hollow section 18 is adapted in its shape to the shape of the head 3, in the embodiment shown, it is a spherical section to accommodate the spherical head 3. Furthermore, the hollow section 18 is configured to encompass the head 3 of the bone anchoring element 1 from the side covering a region including the largest dameter of the head 3.

A plurality of slits 20 are provided in the head receiving portion 17 that are open to the free end 17b. The slits 20 render the head receiving portion 17 flexible so that it can be compressed to clamp and finally lock the head 3 in then hollow internal portion 18 by means of friction. The number and size of the slits 20 is provided depending on the desired flexibility of the head receiving portion 17. The flexibility of the head receiving portion 17 is such that the head 3 of the bone anchoring element can be inserted by expanding the head receiving portion 17 and that it can be clamped by compressing the head receiving portion.

The outer surface of the head receiving portion 17 has a first section 21 with an outer diameter increasing towards the free end 17b, for example, in an outwardly curved or conically widening manner. Adjacent to the first section 21, there is a circumferential groove 22 that is recessed with respect to the first section 21 and that serves for engagement with a portion of the locking ring 8. The groove 22 is shaped so as to allow a disengagement of the locking ring and the groove 22 when moving the locking ring in a direction towards the free end 17b. This is realized, for example, in that the lower wall of the groove is inclined towards the free end 17b.

Adjacent the groove 22, there is a third portion 23 of the head receiving portion with a substantially cylindrical outer surface. The third portion 23 is configured to cooperate with a portion of the locking ring to enhance the clamping effect of the locking ring.

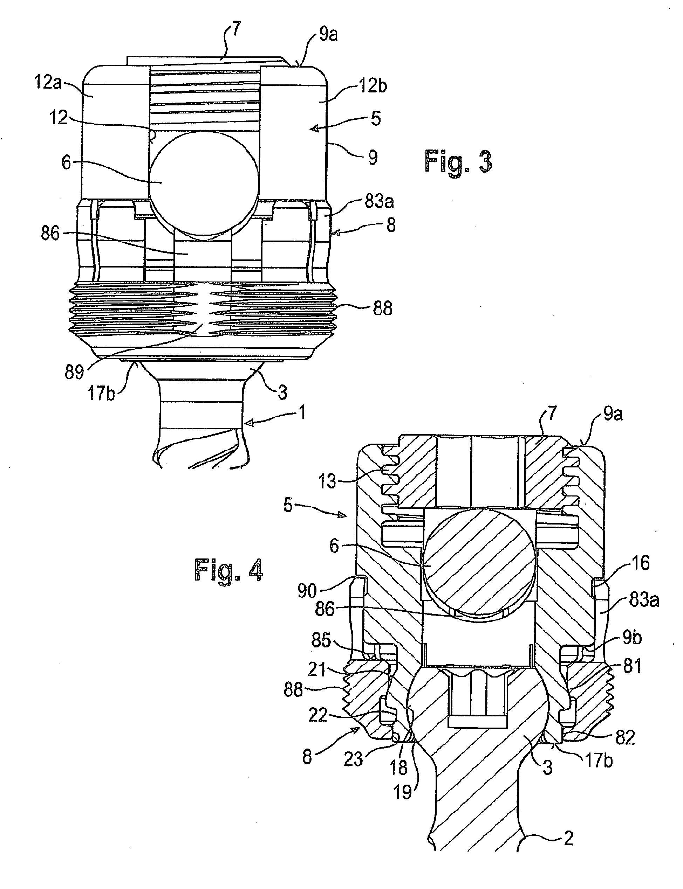

The locking ring will now be described in particular with reference to Figs. 3, 4 and 8 to 10. The locking ring 8 is substantially cylindrical and has an upper end 8a and a lower end 8b. In the mounted state, the upper end 8a is oriented in the direction of the first end 9a of the rod receiving portion and the lower end 8b is oriented in the direction of the free end 17b of the head receiving portion 17. Approximately in the middle of the locking ring, at the inner wall a first portion 81 is provided that cooperates with the first outer surface portion 21 of the head receiving portion 17 to compress the head receiving portion. The first portion 81 may be slightly tapered or may be straight or curved with a curvature directing to the center of the locking ring. Furthermore, at the lower end 8b, the locking ring comprises an inwardly projecting edge 82, the inner diameter of which is smaller than the inner diameter of the other portions of the locking ring. The inwardly projecting edge 82 is configured to engage the groove 22 of the head receiving portion and configured to finally engage the cylindrical portion 23 of the head receiving portion 17.

The locking ring 8 also has a third portion consisting of upwardly extending wall portions 83a that are separated from each other by slits 84. The upwardly extending wall portions 83a are arranged at the outer circumference of an inner circumferential shoulder 85 of the locking ring and render the third portion of the locking ring flexible. The number and size of the slits 84 and the thickness of the wall portions 83a are configured such that a desired flexibility is obtained. At the free end, the wall portions 83a are configured to engage the groove 16 provided on the outer surface of the rod receiving portion 9.

Two projections 86 that are located diametrically opposite to each other, are formed in the second portion 83 of the locking ring. The projections 86 have such a height that they project above the bottom of the substantially U-shaped recess 12 and extend into the cut-outs 15 when the locking ring is in a position where its shoulder 85 abuts the second end 9b of the rod receiving portion. The free end surface 86a of the projections may be curved. The locking ring is arranged around the head receiving portion 17 of the receiving part 5 such that the projections 86 are located at the positions of the recess 12. By means of this, the projections 86 prevent the locking ring 8 from rotating when the rod is not inserted.

The locking ring further comprises on its outer surface portion adjacent the upwardly extending wall portions 83a an engagement portion 88 for engagement with a tool described below. In the first embodiment, the engagement portion 88 is realized by an external thread. The thread extends in axial direction of the locking ring along a portion of the outer surface of the locking ring. The depth of the thread is small so that a violation of surrounding tissue or blood vessels is avoided. In the direction of the rod support provided by the projections 86, just below the projections 86, the thread is interrupted at two opposite thread-free sections 89. The thread may be flattened towards the center of the thread-free sections 89, respectively. The sections 89 serve for applying a distraction or compression tool. The thread-free sections 89 can also be provided at other positions or more than two thread-free sections 89 can be provided.

The receiving part, the locking ring, the inner screw and the bone anchoring element are made of a bio-compatible material, for example, of titanium or stainless steel or a bio-compatible alloy such as Nitinol or bio-compatible plastic material, such as PEEK (Polyetheretherketone). The parts can be made all of the same or of different materials.

The locking ring 8 can assume three main positions. In a first position, the locking ring is engaging with the inwardly projecting edge 82 the groove 22 of the head receiving portion. In this position, the head 3 of the bone anchoring element can be introduced into the internal hollow space 18 from the free end 17b of the head receiving portion. The locking ring 8 is prevented from moving upwards to towards the first end 9a of the rod receiving portion, because it abuts with the shoulder 85 against the second end 9b of the head receiving portion.

In a second main position, the locking ring 8 is shifted towards the free end 17b of the head receiving portion until the flexible wall sections 83a snap with their free ends into the groove 16 of the rod receiving portion. In this position, the head 3 is not yet locked but is prevented from removal out of the internal hollow space 18. The head may be frictionally clamped in this position to such an extent that it is still movable when a force, for example, manually applied force, is applied to overcome the friction force. The head 3 cannot be removed from the head receiving portion 17 in this position.

In a third position of the locking ring, the locking ring 8 is shifted further towards the free end 17b of the head receiving portion such that the head 3 is finally locked. In this position, the head receiving portion is compressed by the locking ring so that the head cannot move and is fixed in its angular position with respect to the receiving part 5. Between the upper end of the flexible wall sections 83a and the upper wall of the groove 16 in the head receiving portion 9 is a gap 90, as shown in Fig. 4. In the locked condition of the head 3, the locking ring 8 cannot be loosened under normal operating conditions.

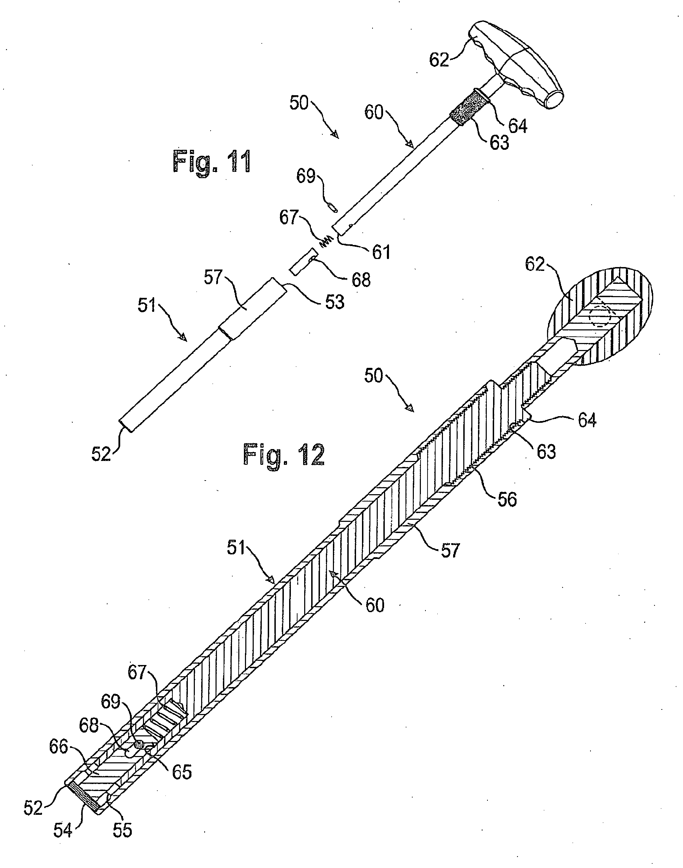

A tool 50 according to a first embodiment will now be described with reference to Figs. 11 and 12.

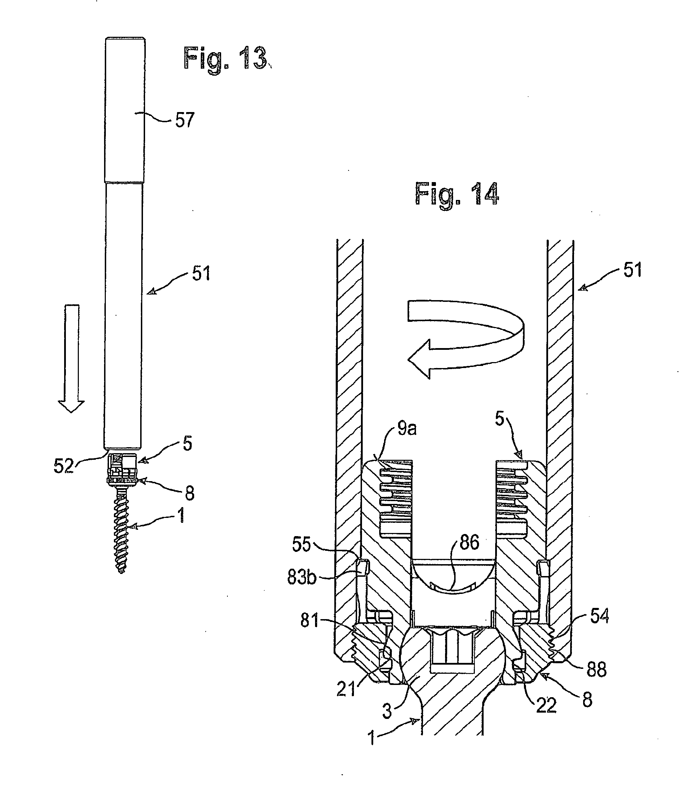

The tool 50 comprises a tubular member 51 with a front end 52 and a rear end 53 opposite to the front end 52. Adjacent the front end 52 the tubular member 51 comprises at its inner wall an engagement portion 54 for engaging the engagement portion 88 of the locking ring. The inner diameter of the tubular member 51 at the front end is therefore such that the tubular member can be placed onto the receiving part 5 and engage the locking ring 8. At a distance from the front end 52 a stop 55 is provided within the tubular member that abuts against the upper edge 8a of the locking ring when the engagement portion 54 engages the threaded portion 88 as shown in Fig. 14. At its rear end 53, the tubular member has an internal threaded portion 56.

The tool 50 further comprises a shaft 60 with a front end 61 and a rear end 62 that is formed as a handle. The shaft 60 has an outer threaded portion 63 at a distance from the handle that cooperates with the internal thread 56 of the tubular member. Adjacent the outer threaded surface portion 63 is a stop 64 that limits the insertion depth of the shaft 60 into the tubular member. The stop 64 can be, for example, a circumferential shoulder. The shaft 60 is insertable into the tubular member 50 and movable therein. The insertion depth can be adjusted by the cooperation of the threads 56 and 63 of the tubular member and the shaft, respectively. As shown in Fig. 12, when the shaft is inserted and screwed into the tubular member until the stop 64 abuts against the rear end 53, the front end 61 of the shaft is at a distance from the front end 52 of the tubular member.

The shaft has at its front end 61 a blind hole 65, in which a post 66 is supported by a spring 67. An end surface 61a of the post 66 projects out of the front end 61 of the shaft and the post 66 is movable in the blind hole 65. The movement of the post 66 in the blind hole 65 is limited in two directions by a stop. The stop is formed by an axially elongated hole 68 that extends through the post 66 and through which a pin 69 is passed. The pin 69 extends through a transverse hole 70 provided in the shaft 60 as shown in particular in Fig. 11. By means of this, the post 66 is moveable between a first position in which the pin is at the end of the elongate hole 68 that faces the spring 67 to a second position, in which the pin 69 is at the end of the elongate hole 68 that faces the front end 61 of the shaft. In the first position, the post 66 projects further outward from the front end 61 and may be biased by partial compression of the spring 67. In the second position, the post 66 is urged towards the end of the blind hole thereby further compressing the spring 67. The spring 67 is shown as a helical spring, but it can be any other spring that fulfils the same purpose. For example, the spring 67 can also be realized by an elastomer cushion.

The function of the tool will be described with reference to Figs. 13 to 24. First, the tubular member 50 is separate from the shaft 60. As shown in Fig. 13, the tubular member is placed over the receiving part 5 until the threaded portion 54 engages the threaded portion 88 of the locking ring 8, as shown in Fig. 14.

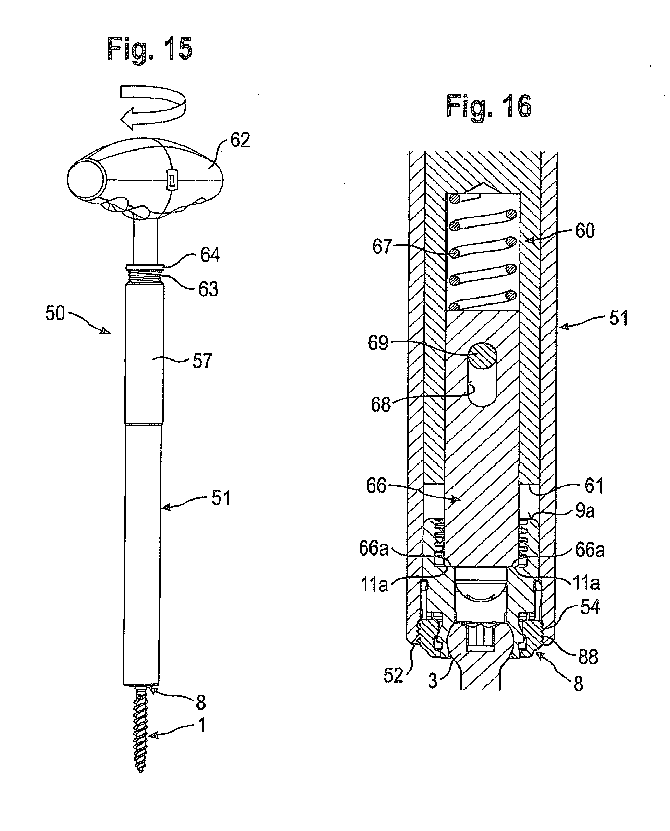

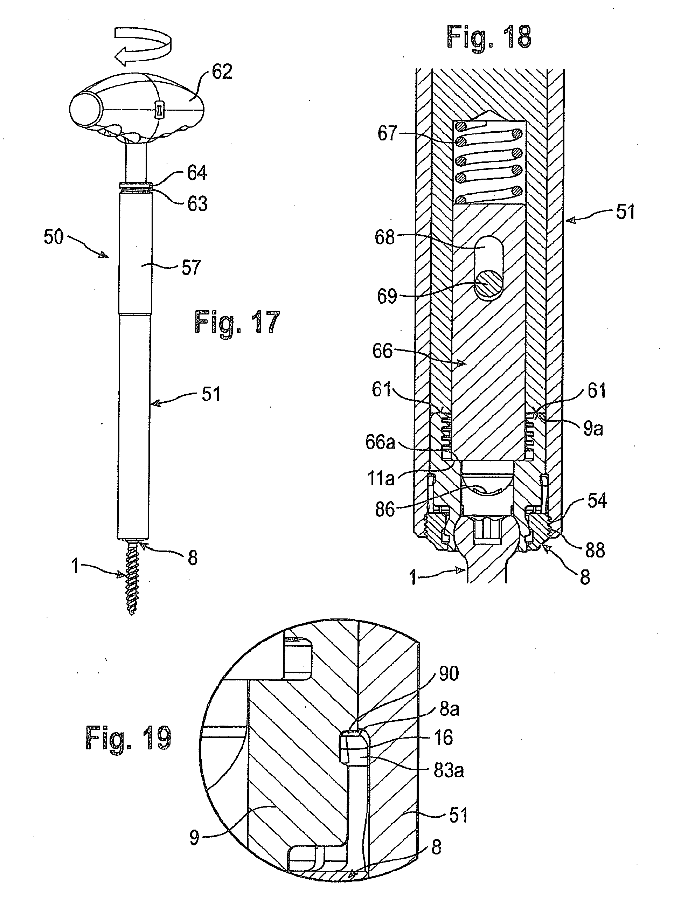

In a next step, shown in Figs. 15 and 16, the shaft 60 is inserted into the tubular member 50 until the front end 66a of the post 66 abuts against the abutment surface 11a in the receiving part 5 (Fig. 16). In this position, the front end 61 of the shaft 60 is spaced apart from the first end 9a of the rod receiving portion 9 of the receiving part 5. The post 66 is in its first position, where the pin 69 faces the spring 67. The shaft 60 is not yet fully introduced into the tubular member 50. As shown in Figs. 17 and 18, the shaft 60 is thereafter further screwed into the tubular member 50 until the front end 61 of the shaft abuts against the first end 9a of the rod receiving portion 9 of the receiving part. Thereby, the counterforce acting onto the post 66 presses the post 66 into the blind hole 65 against the force of the spring 67. In this condition, the locking ring 8 is still in its third position, in which it locks the head. This can be seen in the enlarged representation according to Fig. 19 that shows the gap 90 between the upper end 8a of the locking ring and the wall of the groove 16 facing the upper end 8a.

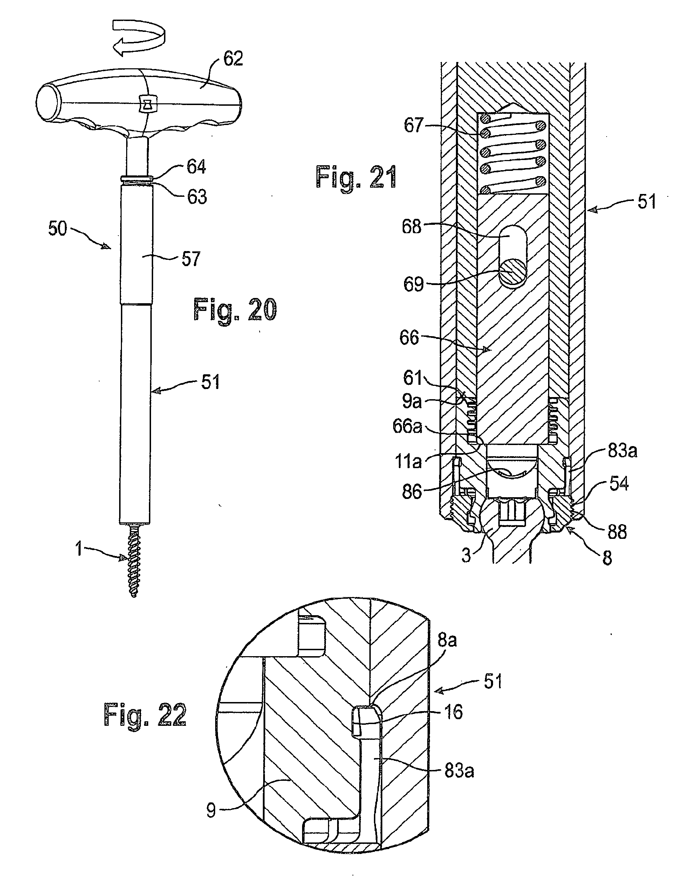

For releasing the locking ring 8 from the locking position, the tubular member 50 is gripped at the gripping portion 57 and held in this position while the shaft 60 is further screwed into the tubular member 50 as shown in Fig. 21. By means of this, the locking ring 8 is drawn upwards until flexible wall section 83a abuts the upper wall of groove 16, as shown in detail in Fig. 22. Hence, the locking ring is now in the second position, where it does not lock the head.

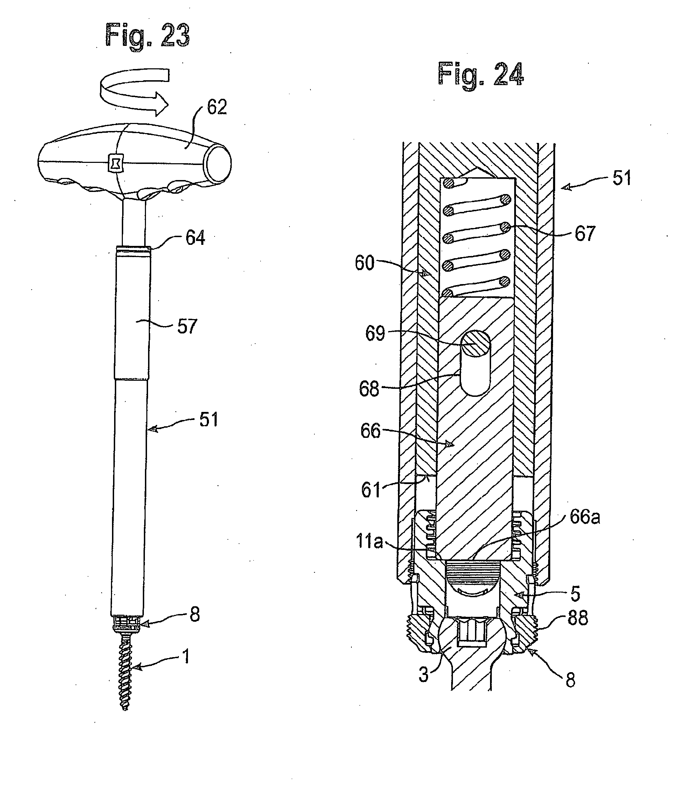

The step of removing the tool is shown in Figs. 23 and 24. The handle 62 is turned into the other direction. The locking ring 8 abuts against the groove 16, therefore, it can not be drawn further upwards. Because the post 66 abuts against the abutment surface 11a of the receiving part, the screwing back of the shaft 60 does not result in simultaneous rotation of the receiving part and the locking ring. Hence, the tool can be removed easily. Then, the angular position between the bone anchoring element and the tool can be readjusted.

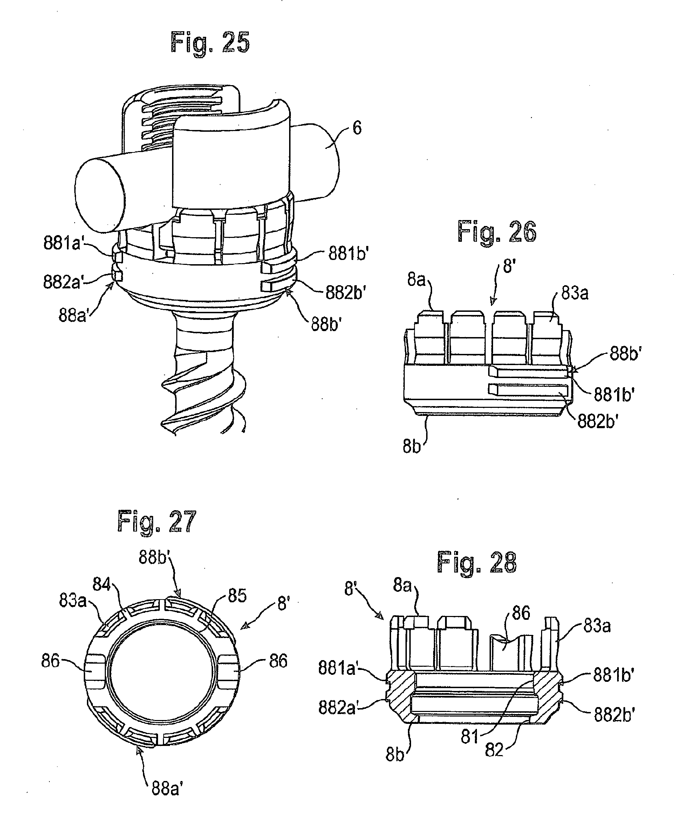

A second embodiment of the bone anchoring device is shown in Figs. 25 to 28. The second embodiment differs from the bone anchoring device according to the first embodiment only by the design of the engagement portion of the locking ring. All other parts are identical or similar to the first embodiment and are marked with the same reference numerals. The description of these parts is not repeated.

The locking ring 8' has an engagement portion for the tool that is in the form of diametrically opposite lying circumferential rib portions 88'. In the embodiment shown, two rib portions 88a' and 88b' that are arranged at the outer surface of the locking ring 8' beneath the elastically deformable wall portions 83a are provided. The rib portions 88a', 88b' extend over a segment of the outer circumference of the locking ring that is approximately a quarter circle or less than a quarter circle. The rib portions 88a', 88b' are arranged at an angle of approximately 45° with respect to the projections 86 that support the rod 6.

Each rib portion 88a', 88b' consists of two circumferentially extending ribs 881a', 882a' that are spaced from each other in an axial direction of the locking ring 8'. The ribs 881a', 882a', 881b', 882b' are slightly inclined towards the second end 8b of the locking ring 8' as can be seen in particular in Fig. 28. The downward inclination of the ribs provide an undercut for the engagement with the tool described below that enhances the safety of the engagement with the tool. In the embodiment shown, two ribs in each rib portion are provided. It shall be noted, however, that one rib for each rib portion may be sufficient. Also, more than two ribs can be provided.

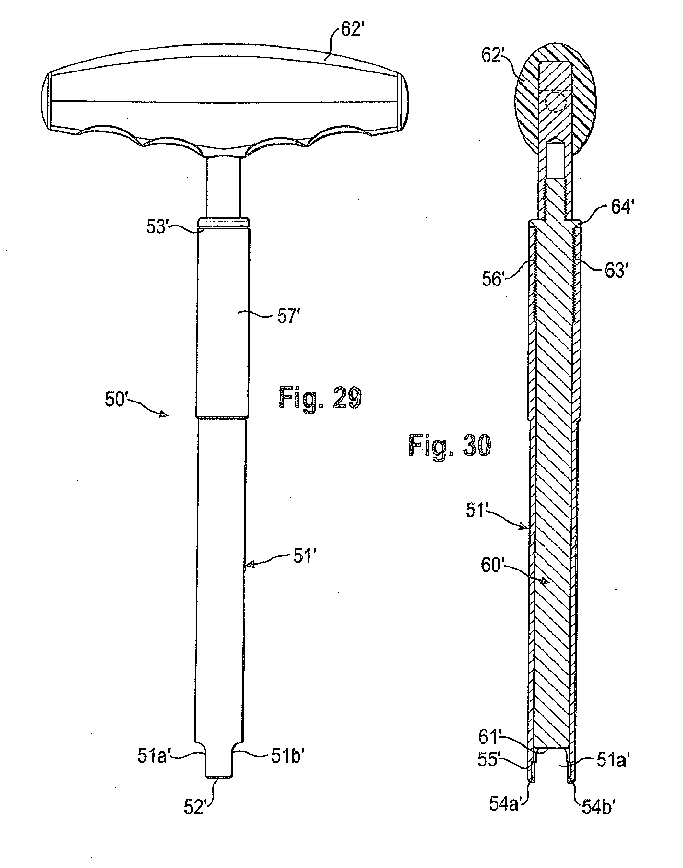

A tool for releasing the locking ring according to a second embodiment will be described referring to Figs. 29 and 30. The tool 50' comprises two substantially rectangular recesses 51 a', 51 b' at its front end 52'. Adjacent the front end 52' engagement portions 54' that correspond to and cooperate with the rib portions 88a', 88b' are located inside the tubular member 51'. The engagement portions 54a', 54b' are rib portions with an inclination away from the front end 52'. At a distance from the front end 52', a stop 55' is provided. The substantially rectangular recesses 51a', 51 b' have a size that is approximately the size of the rib-free portions of the locking ring in a circumferential direction and have a depth in axial direction that is such that when the tubular member 51' is placed onto the receiving portion and engages the locking ring, the rod 6 can pass through the recess 51a' 51b'.

The tubular member 51' also comprises a rear end 53' with an internally threaded portion 56'. At or in the vicinity of the rear end 53', a grip portion 57' is provided for facilitating gripping. The tool 50' further includes a shaft portion 60' with a front end 61' and a rear end 62' that is provided with a handle. At a distance from the rear end 62' an externally threaded portion 63' is provided that cooperates with the internally threaded portion 56' of the tubular member 51' as in the first embodiment. Furthermore, as in the first embodiment, a stop in form of an annular shoulder 64' is provided at a distance from the rear end. The shaft 60' in this embodiment doesn't have the resiliently supported post as in the previous embodiment. The length of the shaft is such that when the shaft is inserted and screwed into the tubular member until the stop 64' abuts against the rear end 53' of the tubular member, the recesses 51a', 51b' at the front end 52' can cover the receiving part and the rod inserted therein.

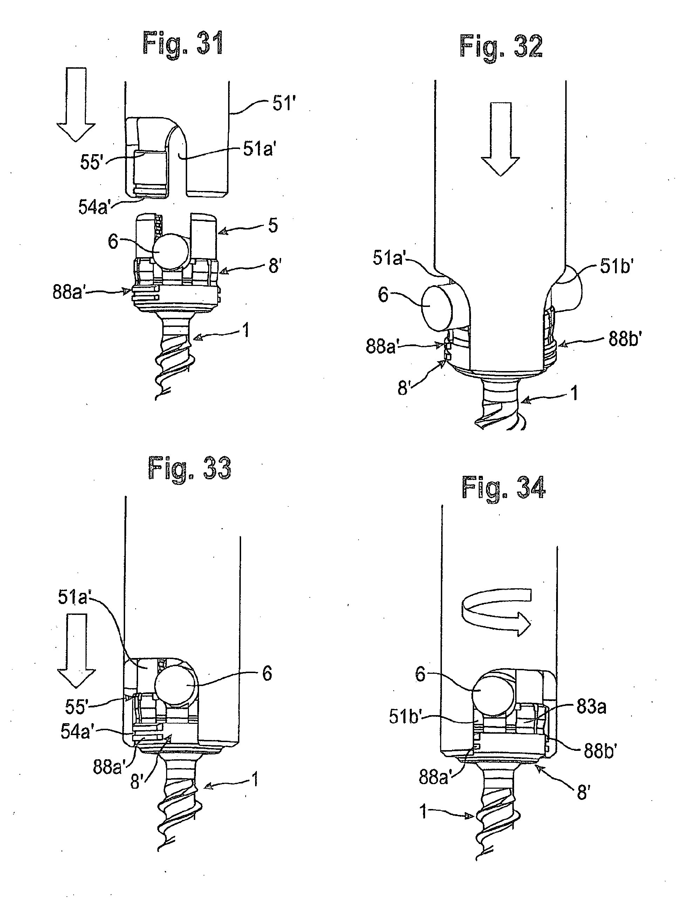

The tool 50' according to the second embodiment can be applied when the rod is inserted and the inner screw is removed. As shown in Fig. 31, the tool is oriented such that the engagement portion in form of the ribs 54a', 54b' are placed at the rib-free portions of the locking ring 8'. The rod 6 then abuts against one side of the recesses 51a',51b'. The tubular member 50' is shifted downwards until the stop 55' abuts against the upper edge 8a of the locking ring 8'. Thereafter, as shown in Figs. 32 to 34, the tubular member is rotated so that the engagement portions 54a', 54b' inside the tubular member engage the rib portions 88a', 88b' on the outer surface of the locking ring. Because the recesses 51a', 51b' are sufficiently large in a circumferential direction, the tubular member 50' can be rotated such that the rod abuts against the other side of the recess 51a', 51b', as shown in Fig. 34.

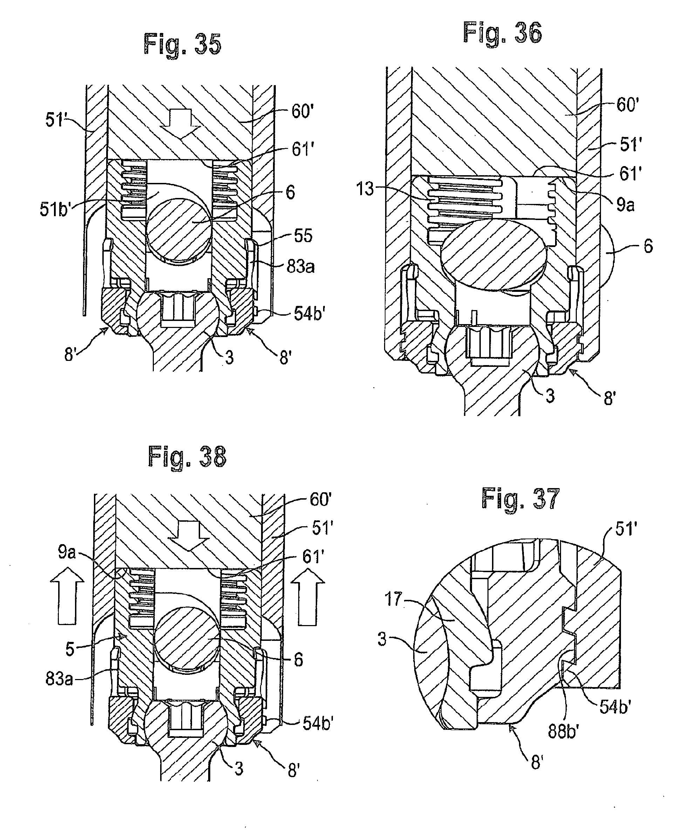

Thereafter, as shown in Figs. 35 to 37, the shaft 60' is screwed into the tubular member 50' until its front end 61' abuts against the first end 9a of the receiving part. In a next step, as shown in Fig. 38, the shaft is further moved into the tubular member while the tubular member is held at the gripping portion 57'. By means of this, the locking ring is pressed upwards and is released from the locking position.

For the removal of the tool 50', the shaft 60' is slightly screwed back, then the tubular member 51' is rotated until the engagement portions of the tubular member no longer engage the engagement portions at the locking ring. The tool can then be removed by pulling it upwards. It shall be noted that modifications of the embodiments shown are pivotable. For example, it is possible that the tool according to the second embodiment also includes a post that is supported by a spring. Also, the tool according to the second embodiment may have a threaded engagement portion for engaging a threaded locking ring according to the first embodiment. The tool of the first embodiment may be provided without the post.

For the engagement at the locking ring other modifications are also conceivable. For example, it may be possible to have engagement portions with another shape.

The bone anchoring device can be provided in a modified form. For example, the head of the bone anchoring element can have any other shape, such as, for example, a cylindrical shape, whereby a monoaxial bone screw is provided allowing rotation of the screw element with respect to the receiving part around a single axis. The head can also be conically shaped or otherwise shaped and the internal hollow section of the head receiving portion is adapted to this shape. In a further modification, the flexibility of the head receiving portion is based on properties of the material, for example of a plastic material, and the slits may be fully or partly omitted.

The projections of the locking ring that engage the rod can have another shape. The surface of the free end can be flat or can be otherwise shaped. The projections can be also omitted.

The head receiving portion can have an inclined open end or can be otherwise asymmetric to allow a greater angulation of the head in one direction.

The outer surface of the head receiving portion and the inner surface of the locking ring can have other shapes that allow a compression of the locking ring by means of an increasing force when the locking ring is shifted downward.

The locking ring can also have another design. For example, the locking ring can be formed without the flexible wall sections.

## Claims

1. Bone anchoring device comprising
a bone anchoring element (1) having a shank (2) for anchoring in the bone and a head (3);
a receiving part (5) for coupling a rod to the bone anchoring element, wherein the receiving part comprises
a rod receiving portion (9) with a first end (9a) and a second end (9b) and a U-shaped recess (12) for receiving the rod, the recess extending from the first end in the direction of the second end thereby forming two free legs (12a, 12b) and
a head receiving portion (17) at the side of the second end opposite to the first end for accommodating the head (3), the head receiving portion having a free end (17b) and being flexible so as to allow introduction and clamping of the head;
a locking ring (8, 8') arranged around the head receiving portion;
wherein the locking ring can assume a locking position in which the head is locked by compression of the head receiving portion of the receiving part;
wherein the locking ring (8, 8') comprises an outer surface portion with an engagement structure (88, 88') for engagement with a tool, **characterized in that** the engagement structure comprises a plurality of ribs (88, 88') extending in a circumferential direction of the locking ring.

2. The bone anchoring device of claim 1, wherein the ribs (88, 88') have interruptions in a circumferential direction.

3. The bone anchoring device of claim 1, wherein the ribs (88') comprise at least two ribs (88a', 88b') that have a distance from each other in an axial direction of the locking ring and that extend substantially perpendicular to a central axis of the locking ring.

4. The bone anchoring device of one of claims 1 to 3, wherein the ribs (88) are formed by a thread with a thread axis being the central axis of the locking ring.

5. The bone anchoring device of one of claims 1 to 4, wherein the locking ring (8, 8') has two projections (86) arranged at 180° offset from each other for engagement with the rod.

6. The bone anchoring device of claim 5, wherein the ribs (88, 88') arc grouped in two groups to the left and right of the projections.

7. The bone anchoring device of claim 5, wherein the groups are arranged a asymmetrical with respect to the projections (86).

8. The bone anchoring device of one of claims 1 to 7, wherein the head receiving portion has an exterior surface (21) with a tapered or outwardly curved portion and the locking ring has an interior surface (81) with a tapered or an inwardly curved portion which are configured to cooperate such that the head is clamped when the locking ring is moved towards the free end (17b).

9. System comprising a tool (50, 50') for releasing the locking of a head in a bone anchoring device according to one of claims 1 to 8 and the bone anchoring device according to one of claims 1 to 8, the tool comprising
a tubular member (51, 51') having a front end (52, 52') and an engagement portion (54, 54a', 54b') at an inner wall of the front end for engaging the engagement structure (88, 88') of the locking ring (8, 8'),
a shaft (60, 60') insertable into the tubular member (51,51') and movable relative to the tubular member in an axial direction, the shaft having a front end (61, 61'),
wherein the front end (61, 61') of the shaft abuts against a first portion (9a) of the receiving part when the engagement portion (54, 54a', 54b') of the front end (52, 52') of the tubular member engages the engagement portions (88, 88') of the locking ring, and wherein in this condition the tool moves the shaft relative to the tubular member so as to move the locking ring to release the locking of the head.

10. System of claim 9, wherein the front end (61) of the shaft has a coaxial bore (65) and wherein a post (66) is provided within the bore that extends out of the bore and is movable relative to the shaft, and wherein the post is preferably elastically supported.

11. System of claim 10, wherein the path of movement of the post (66) relative to the shaft (60) is limited by at least one stop (68, 69).

12. System of one of claims 10 to 11, wherein the post (66) is configured to abut against a second portion (11a) of the receiving part (5).

13. System of one of claims 9 to 12, wherein at the front end (51') of the tubular member two recesses (51a',51 b') located opposite to each other are provided that are configured to allow the rod to pass therethrough.

14. System of claim 13, wherein the recesses (51a', 51b') are substantially rectangular with a width configured to allow rotation of the tubular member so as to engage the engagement portions when the rod is inserted.

15. System of claim 13 or 14, wherein at a distance from the front end (52, 52') of the tubular member an abutment (55, 55') is provided for abutting against an upper edge (8a) of the locking ring (8, 8').

16. System of one of claims 9 to 15, wherein the tubular member (51, 51') has a grip portion (57, 57') and preferably the shaft (60, 60') has a grip or a handle portion.

## Patentansprüche

1. Knochenverankerungsvorrichtung umfassend
ein Knochenverankerungselement (1) mit einem Schaft (2) zum Verankern im Knochen und einen Kopf (3);
ein Aufnahmeteil (5) zum Koppeln eines Stabes an das Knochenverankerungselement, wobei das Aufnahmeteil umfasst
einen Stabaufnahmeabschnitt (9) mit einem ersten Ende (9a) und einem zweiten Ende (9b) und einer U-förmigen Ausnehmung (12) zum Aufnehmen des Stabs, wobei die Ausnehmung sich von dem ersten Ende in Richtung des zweiten Endes erstreckt und dabei zwei freie Schenkel (12a, 12b) bildet und
einen Kopfaufnahmeabschnitt (17) an der Seite des zweiten Endes gegenüberliegend dem ersten Ende zum Aufnehmen des Kopfes (3), wobei der Kopfaufnahmeabschnitt ein freies Ende (17b) aufweist und flexibel ist so dass er ein Einführen und Klemmen des Kopfes erlaubt;
einen Verriegelungsring (8, 8'), der um den Kopfaufnahmeabschnitt befestigt ist;
wobei der Verriegelungsring (8, 8') eine Verriegelungsposition einnehmen kann in der der Kopf durch Zusammendrücken des Kopfaufnahmeabschnitts des Aufnahmeteils verriegelt ist;
wobei der Verriegelungsring (8, 8') einen äußeren Oberflächenabschnitt mit einer Eingriffsstruktur (88, 88') umfasst zum in Eingriff bringen mit einem Werkzeug, **dadurch gekennzeichnet, dass**
die Eingriffsstruktur eine Mehrzahl von Rippen (88, 88') umfasst, die sich in einer Umfangsrichtung des Verriegelungsringes erstrecken.

2. Knochenverankerungsvorrichtung nach Anspruch 1, wobei die Rippen (88, 88') in einer Umfangsrichtung Unterbrechungen aufweisen.

3. Knochenverankerungsvorrichtung nach Anspruch 1, wobei die Rippen (88') wenigstens zwei Rippen (88a', 88b') umfassen die in einer axialen Richtung des Verriegelungsringes eine Entfernung zueinander haben und die sich im Wesentlichen senkrecht zu einer Zentralachse des Verriegelungsringes erstrecken.

4. Knochenverankerungsvorrichtung nach einem der Ansprüche 1 bis 3, wobei die Rippen (88) durch ein Gewinde gebildet sind mit einer Gewindeachse, die die Zentralachse des Verriegelungsringes ist.

5. Knochenverankerungsvorrichtung nach einem der Ansprüche 1 bis 4, wobei der Verriegelungsring (8, 8') zwei Vorsprünge (86) aufweist die mit 180° Versatz voneinander angeordnet sind zum in Eingriff bringen mit dem Stab.

6. Knochenverankerungsvorrichtung nach Anspruch 5, wobei die Rippen (88, 88') in zwei Gruppen rechts und links der Vorsprünge gruppiert sind.

7. Knochenverankerungsvorrichtung nach Anspruch 5, wobei die Gruppen asymmetrisch in Bezug auf die Vorsprünge (86) angeordnet sind.

8. Knochenverankerungsvorrichtung nach einem der Ansprüche 1 bis 7, wobei der Kopfaufnahmeabschnitt eine Außenfläche (21) mit einem zulaufenden oder nach außen gekrümmten Abschnitt aufweist und der Verriegelungsring eine Innenoberfläche (81) mit einem zulaufenden oder nach innen gekrümmten Abschnitt aufweist die ausgebildet sind so zusammenzuwirken, dass der Kopf geklemmt wird wenn der Verriegelungsring in Richtung des freien Endes (17b) bewegt wird.

9. System umfassend ein Werkzeug (50, 50') zum Lösen der Verriegelung des Kopfes in einer Knochenverankerungsvorrichtung nach einem der Ansprüche 1 bis 8 und die Knochenverankerungsvorrichtung nach einem der Ansprüche 1 bis 8, wobei das Werkzeug umfasst
ein röhrenförmiges Teil (51, 51') mit einem Vorderende (52, 52') und einem Eingriffsabschnitt (54, 54a', 54b') an einer inneren Wand des Vorderendes zum in Eingriff bringen der Eingriffsstruktur (88, 88') des Verriegelungsringes (8, 8'),
einen Schaft (60, 60') der in das röhrenförmige Teil (51, 51') einführbar und relativ zu dem röhrenförmigen Teil in eine axiale Richtung bewegbar ist, wobei der Schaft ein Vorderende (61, 61') aufweist,
wobei das Vorderende (61, 61') des Schaftes an einen ersten Abschnitt (9a) des Aufnahmeteils anstößt wenn der Eingriffsabschnitt (54, 54a', 54b') des Vorderendes (52, 52') des röhrenförmigen Teils mit den Eingriffsabschnitten (88, 88') des Verriegelungsringes in Eingriff gelangt, und wobei das Werkzeug in diesem Zustand den Schaft relativ zu dem röhrenförmigen Teil so bewegt, dass es den Verriegelungsring so bewegt, dass es die Verriegelung des Kopfes löst.

10. System nach Anspruch 9, wobei das Vorderende (61) des Schaftes eine koaxiale Bohrung (65) aufweist und wobei ein Stift (66) in der Bohrung vorgesehen ist der aus der Bohrung heraus steht und in Bezug zum Schaft bewegbar ist, und wobei der Stift bevorzugt elastisch gelagert ist.

11. System nach Anspruch 10, wobei der Bewegungsverlauf des Stiftes (66) bezüglich des Schaftes (60) durch wenigstens einen Anschlag (68, 69) begrenzt ist.

12. System nach einem der Ansprüche 10 bis 11, wobei der Stift (66) ausgebildet ist an einen zweiten Abschnitt (11a) des Aufnahmeteils (5) anzustoßen.

13. System nach einem der Ansprüche 9 bis 12, wobei an dem Vorderende (51') des röhrenförmigen Teils zwei Ausnehmungen (51a', 51b') einander gegenüberliegend angeordnet vorgesehen sind, die ausgebildet sind den Stab dort hindurch zu lassen.

14. System nach Anspruch 13, wobei die Ausnehmungen (51a', 51b') im Wesentlichen rechteckig sind mit einem Querschnitt der ausgebildet ist eine Rotation des röhrenförmigen Teiles zu erlauben, so dass sie mit den Eingriffsabschnitten in Eingriff gelangen wenn der Stab eingesetzt ist.

15. System nach einem der Ansprüche 13 oder 14, wobei in einer Entfernung von dem Vorderende (52, 52') des röhrenförmigen Teils ein Anschlag (55, 55') vorgesehen ist zum Anstoßen an einen oberen Rand (8a) des Verriegelungsringes (8, 8').

16. System nach einem der Ansprüche 9 bis 15, wobei das röhrenförmige Teil (51, 51') einen Griffabschnitt (57, 57') aufweist und vorzugsweise der Schaft (60, 60') einen Griff- oder einen Greifabschnitt aufweist.

## Revendications

1. Dispositif d'ancrage osseux comprenant
un élément d'ancrage osseux (1) ayant une bande de jambe (2) pour l'ancrage dans l'os et une tête (3) ;
une partie de réception (5) pour coupler une tige à l'élément d'ancrage osseux, dans lequel la partie de réception comprend
une partie de réception de tige (9) avec une première extrémité (9a) et une deuxième extrémité (9b) et un évidement en forme de U (12) pour recevoir la tige, l'évidement s'étendant à partir de la première extrémité dans la direction de la deuxième extrémité formant ainsi deux jambes libres (12a, 12 b) et
une partie de réception de tête (17) au niveau du côté de la deuxième extrémité opposée à la première extrémité pour recevoir la tête (3), la partie de réception de tête ayant une extrémité libre (17b) et étant flexible de manière à permettre l'introduction et le serrage de la tête ;
un anneau de verrouillage (8, 8') agencé autour de la partie de réception de tête ;
dans lequel l'anneau de verrouillage peut adopter une position de verrouillage dans laquelle la tête est verrouillée par compression de la partie de réception de tête de la partie de réception ;
dans lequel l'anneau de verrouillage (8, 8') comprend une partie de surface externe avec une structure d'engagement (88, 88') pour l'engagement avec un outil, **caractérisé en ce que**
la structure d'engagement comprend une pluralité de nervures (88, 88') s'étendant dans une direction circonférentielle de l'anneau de verrouillage.

2. Dispositif d'ancrage osseux de la revendication 1, dans lequel les nervures (88, 88') ont des interruptions dans une direction circonférentielle.

3. Dispositif d'ancrage osseux de la revendication 1, dans lequel les nervures (88') comprennent au moins deux nervures (88a', 88b') qui sont espacées d'une distance dans une direction axiale de l'anneau de verrouillage et qui s'étendent de manière substantiellement perpendiculaire à un axe central de l'anneau de verrouillage.

4. Dispositif d'ancrage osseux de l'une des revendications 1 à 3, dans lequel les nervures (88) sont formées par un filetage avec un axe de filetage étant l'axe central de l'anneau de verrouillage.

5. Dispositif d'ancrage osseux de l'une des revendications 1 à 4, dans lequel l'anneau de verrouillage (8, 8') a deux saillies (86) agencées de manière à être décalées de 180° pour l'engagement avec la tige.

6. Dispositif d'ancrage osseux de la revendication 5, dans lequel les nervures (88, 88') sont regroupées en deux groupes à gauche et à droite des saillies.

7. Dispositif d'ancrage osseux de la revendication 5, dans lequel les groupes sont agencés de manière asymétrique par rapport aux saillies (86).

8. Dispositif d'ancrage osseux de l'une des revendications 1 à 7, dans lequel la partie de réception de tête a une surface extérieure (21) avec une partie effilée ou courbée vers l'extérieur et l'anneau de verrouillage a une surface intérieure (81) avec une partie effilée ou courbée vers l'intérieur qui sont configurées pour coopérer de sorte que la tête soit serrée lorsque l'anneau de verrouillage se déplace vers l'extrémité libre (17b).

9. Système comprenant un outil (50, 50') pour libérer le verrouillage d'une tête dans un dispositif d'ancrage osseux selon l'une des revendications 1 à 8 et le dispositif d'ancrage osseux selon l'une des revendications 1 à 8, l'outil comprenant
un élément tubulaire (51, 51') ayant une extrémité avant (52, 52') et une partie d'engagement (54, 54a', 54b') au niveau d'une paroi interne de l'extrémité avant pour s'engager avec la structure d'engagement (88, 88') de l'anneau de verrouillage (8, 8'),
un arbre (60, 60') pouvant être inséré dans l'élément tubulaire (51, 51') et mobile par rapport à l'élément tubulaire dans une direction axiale, l'arbre ayant une extrémité avant (61, 61'),
dans lequel l'extrémité avant (61, 61') de l'arbre vient en butée contre une première partie (9a) de la partie de réception lorsque la partie d'engagement (54, 54a', 54b') de l'extrémité avant (52, 52') de l'élément tubulaire s'engage avec les parties d'engagement (88, 88') de l'anneau de verrouillage, et dans lequel, dans cette condition, l'outil déplace l'arbre par rapport à l'élément tubulaire de manière à déplacer l'anneau de verrouillage pour libérer le verrouillage de la tête.

10. Système de la revendication 9, dans lequel l'extrémité avant (61) de l'arbre a un alésage coaxial (65) et dans lequel un montant (66) est prévu à l'intérieur de l'alésage qui s'étend à l'extérieur de l'alésage et est mobile par rapport à l'arbre, et dans lequel le montant est de préférence supporté de manière élastique.

11. Système de la revendication 10, dans lequel le trajet de déplacement du montant (66) par rapport à l'arbre (60) est limité par au moins un élément d'arrêt (68, 69).

12. Système de l'une des revendications 10 à 11, dans lequel le montant (66) est configuré pour venir en butée contre une deuxième partie (11a) de la partie de réception (5).

13. Système de l'une des revendications 9 à 12, dans lequel au niveau de l'extrémité avant (51') de l'élément tubulaire, deux évidements (51a', 51b') situés à l'opposé l'un de l'autre sont prévus qui sont configurés pour permettre à la tige de passer à travers ceux-ci.

14. Système de la revendication 13, dans lequel les évidements (51a', 51b') sont substantiellement rectangulaires avec une largeur configurée pour permettre la rotation de l'élément tubulaire de manière à s'engager avec les parties d'engagement lorsque la tige est insérée.

15. Système de la revendication 13 ou 14, dans lequel à une distance de l'extrémité avant (52, 52') de l'élément tubulaire, une butée (55, 55') est prévue pour venir en butée contre un bord supérieur (8a) de l'anneau de verrouillage (8, 8').

16. Système de l'une des revendications 9 à 15, dans lequel l'élément tubulaire (51, 51') a une partie de préhension (57, 57') et de préférence l'arbre (60, 60') a une partie de préhension ou de poignée.
